(19) Europäisches Patentamt
European Patent Office
Office européen des brevets

(11) Veröffentlichungsnummer: **0 009 679**
**B1**

# (12) EUROPÄISCHE PATENTSCHRIFT

(45) Veröffentlichungstag der Patentschrift: **21.04.82**

(51) Int. Cl.³: **C 07 C 143/38**, D 06 L 3/12, H 01 S 3/20

(21) Anmeldenummer: **79103409.3**

(22) Anmeldetag: **12.09.79**

(54) **Fluoreszenzfarbstoffe der Stilbenreihe, Verfahren zu deren Herstellung und ihre Verwendung zum Weisstönen organischer Materialien sowie als Laserfarbstoffe.**

(30) Priorität: **23.09.78 DE 2841519**

(43) Veröffentlichungstag der Anmeldung: **16.04.80 Patentblatt 80/8**

(45) Bekanntmachung des Hinweises auf die Patenterteilung: **21.04.82 Patentblatt 82/16**

(84) Benannte Vertragsstaaten: **CH DE FR GB**

(56) Entgegenhaltungen: **FR-A-1 576 018**

(73) Patentinhaber: **BAYER AG, Zentralbereich Patente, Marken und Lizenzen, D-5090 Leverkusen 1, Bayerwerk (DE)**

(72) Erfinder: **Schieder, Rudolf, Dr., Deutscher Ring 6, D-5030 Hürth (DE)**
Erfinder: **Telle, Helmut, Brabanter Strasse 17, D-5000 Koeln 1 (DE)**
Erfinder: **Raue, Roderich, Dr., Berta-von Suttner-Strasse 48, D-5090 Leverkusen (DE)**
Erfinder: **Brinkwerth, Wolfgang, Dr., Sanderstrasse 89a, D-5060 Bergisch-Gladbach (DE)**

EP 0 009 679 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Fluoreszenzfarbstoffe der Stilbenreihe, Verfahren zu deren Herstellung und ihre Verwendung zum Weißtönen organischer Materialien sowie als Laserfarbstoffe

Gegenstand der Erfindung sind Fluoreszenzfarbstoffe, Verfahren zu ihrer Herstellung und ihre Verwendung zum Weißtönen organischer Materialien sowie als Laserfarbstoffe.

Die neuen Farbstoffe entsprechen der Formel

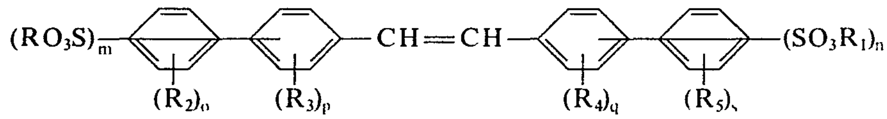

(I)

worin

| | |
|---|---|
| R und $R_1$ | unabhängig voneinander für Wasserstoff, ein salzbildendes Kation, einen Alkylrest mit 1 bis 8 Kohlenstoffatomen oder einen Aralkylrest, |
| $R_2$ bis $R_5$ | unabhängig voneinander für Wasserstoff, Alkyl, Trifluormethyl, Alkoxy, Aralkoxy, Alkenyloxy, Halogen, die Carboxyl-, Cyan-, Alkylsulfon-, Arylsulfon-, Carbonamid-, Sulfonamid- oder die Carbonsäureestergruppe stehen, |
| m und n | unabhängig voneinander 0, 1 und 2 bedeuten, wobei die Summe von m+n mindestens gleich 2 sein muß, und |
| o, p, q und r | unabhängig voneinander für 0, 1 und 2 stehen. |

Als salzbildende Kationen kommen ein- oder zweiwertige Metalle, wie Natrium, Kalium, Lithium, Magnesium, Calcium, Barium, Mangan und Zink in Frage sowie Ammoniumsalze und deren Substitutionsprodukte, die man durch Umsetzung der zugrunde liegenden Säuren mit Mono-, Di- und Trimethylamin, Mono-, Di- und Triethylamin, Mono-, Di- und Triethanolamin, Methyldiethanolamin, Ethyldiethanolamin, Dimethylethanolamin, Diethylethanolamin, Mono-, Di- und Triisopropanolamin, Methyldiisopropanolamin, Ethyldiisopropanolamin, Dimethylisopropanolamin, n-Butylamin, sek. Butylamin, Dibutylamin, Diisobutylamin, Triethoxyethanolamin, Pyridin, Morpholin oder Piperidin erhält.

Bevorzugte Stilbenverbindungen entsprechen der Formel

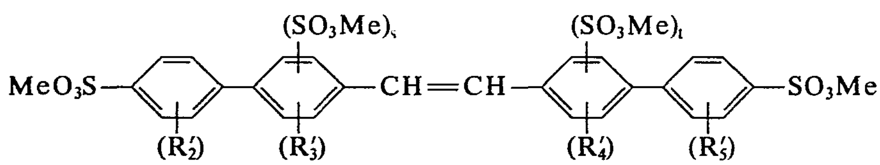

(Ia)

worin

| | |
|---|---|
| Me | für Wasserstoff, Natrium, Kalium oder den gegebenenfalls substituierten Ammoniumrest, |
| $R_2'$ bis $R_5'$ | unabhängig voneinander für Wasserstoff, $C_1$- bis $C_4$-Alkyl, Benzyloxy, Cyan sowie eine Carboxy-, Carbonsäureester- oder Carbonamidgruppe stehen und |
| s und t | 0 oder 1 bedeuten. |

Eine weitere bevorzugte Gruppe entspricht der Formel

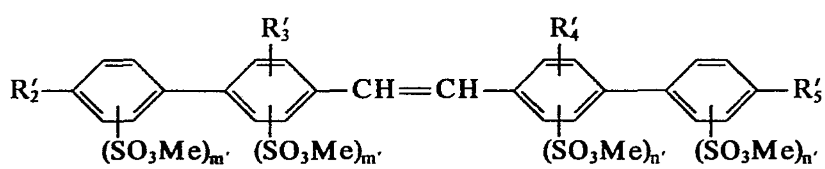

(Ib)

worin

Me und $R_2'$ bis $R_5'$ die gleiche Bedeutung wie in Formel Ia haben und
m' und n' für 0 oder 1 stehen, wobei die Summe von m' und n' mindestens 2 sein muß.

Von besonderer Bedeutung für die Verwendung als Laserfarbstoff ist die Verbindung der Formel

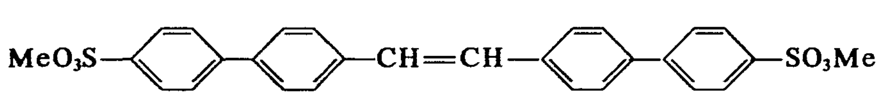

(Ic)

in welcher

Me die gleiche Bedeutung wie in Formel Ia hat.

Die erfindungsgemäßen Stilbenverbindungen lassen sich in an sich bekannter Weise durch Kondensation einer Verbindung der Formel

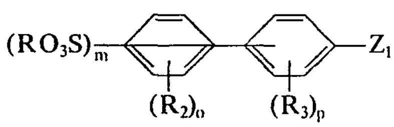

(II)

mit einer Verbindung der Formel

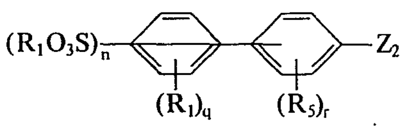

(III)

worin
die Reste R bis $R_5$ und die Indizes m, n, o, p, q, r die gleiche Bedeutung wie in Formel I haben und $Z_1$ und $Z_2$ wechselseitig für die Aldehydgruppe oder einen Rest der Formeln

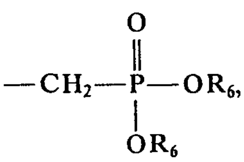, 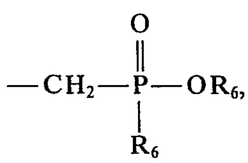, 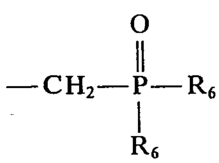 oder 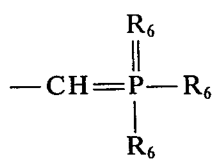

$R_6$ = Alkyl $C_{1-6}$, Aryl, Aralkyl, Cycloalkyl
stehen,

in Gegenwart einer stark basischen Alkaliverbindung und in Gegenwart eines vorzugsweise hydrophilen, stark polaren Lösungsmittels herstellen.

Geeignete Lösungsmittel sind beispielsweise Toluol; Xylol; Chlorbenzol; Alkohole, wie Ethanol; Ethylenglykolmonomethylether; vorzugsweise jedoch N-Methylpyrrolidon; Dimethylformamid; Diethylformamid; Dimethylacetamid oder Dimethylsulfoxid.

Die Temperatur, bei welcher die Umsetzung durchgeführt wird, kann in weiten Grenzen schwanken. Sie wird bestimmt

a) durch die Beständigkeit des verwendeten Lösungsmittels gegenüber den Reaktionsteilnehmern, insbesondere gegenüber den stark basischen Alkaliverbindungen,
b) durch die Reaktivität der Kondensationspartner und
c) durch die Wirksamkeit der Kombination Lösungsmittel/Base als Kondensationsmittel.

Vorzugsweise liegt sie etwa im Bereich von 30°C bis 60°C, doch können in vielen Fällen schon befriedigende Resultate bei Raumtemperatur (ca. 20°C) einerseits oder andererseits bei Temperaturen von 100°C, ja sogar bei der Siedetemperatur des Lösungsmittels erreicht werden, wenn dies aus Gründen der Zeitersparnis oder dadurch erwünscht ist, ein weniger aktives, aber billigeres Kondensationsmittel einzusetzen. Grundsätzlich sind somit auch Reaktionstemperaturen von 10 bis 180°C möglich.

Als stark basische Alkaliverbindungen kommen vor allem die Hydroxide, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen, wie Phenyl-Lithium, oder stark basische Amine (einschließlich Ammoniumbasen, z. B. Trialkylammoniumhydroxide) mit Erfolg verwendet werden.

Die als Ausgangsstoffe benötigten Phosphorverbindungen der Formel II bzw. III werden in an sich bekannter Weise erhalten, indem man Halogenmethylverbindungen, vorzugsweise Chlormethyl- oder Brommethylverbindungen der Formeln

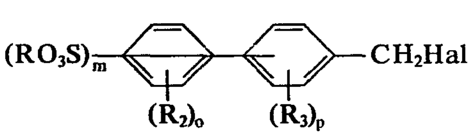

(IV)

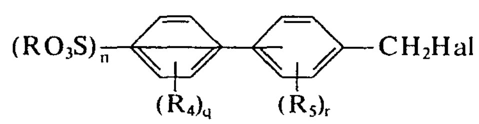

(V)

in denen

die Reste R bis $R_5$ und die Indizes m, n, o, p, q und r die gleiche Bedeutung wie in Formel I haben und Hal für Chlor oder Brom steht,

mit Phosphorverbindungen der Formeln

$(R_6O)_3P$, $R_6P(OR_6)_2$, $(R_6)_2POR_6$, $P(R_6)_3$, in denen

$R_6$ die oben angegebene Bedeutung hat und wobei an Sauerstoff gebundene Reste $R_6$ vorzugsweise niedrige Alkylgruppen, unmittelbar an Phosphor gebundene Reste $R_6$ dagegen vorzugsweise Arylreste sind,

umsetzt.

Die Halogenmethylverbindungen der Formeln IV und V lassen sich aus der entsprechenden Methylverbindung gemäß der in der DE-PS 234 913 beschriebenen Arbeitsweise durch Umsetzung mit Phosphorpentachlorid und Chlor und anschließende Verseifung der Sulfochloride herstellen oder durch Bromierung mit N-Bromsuccinimid gemäß den Angaben der DE-OS 2 262 340. Die Brommethylgruppe läßt sich auch durch Umsetzung des entsprechenden Diphenylderivats mit Paraformaldehyd und Natriumbromid in Schwefelsäure-Eisessig-Mischung nach der in der DE-OS 2 262 340 beschriebenen Arbeitsweise einführen. Ein weiterer Weg zur Herstellung der Halogenmethylverbindungen der Formeln IV und V besteht in der Reduktion der Biphenylaldehydsulfonsäuren mit Natriumborhydrid zu den Hydroxymethylbiphenylsulfonsäuren, der Überführung in die Halogenmethylbiphenylsulfochloride mit Thionylchlorid oder Phosphorpentachlorid und der anschließenden Veresterung der Sulfochloride zu den Sulfonsäureestern.

Geeignete Halogenmethylverbindungen der Formeln IV und V sind:

4-Chlormethylbiphenyl-4'-sulfonsäureethylester,
4-Brommethylbiphenyl-4'-sulfonsäuremethylester,
4-Chlormethylbiphenyl-3-sulfonsäureethylester,
4-Chlormethylbiphenyl-3,4'-disulfonsäurediethylester,
4-Brommethyl-4'-methylbiphenyl-3,3'-disulfonsäurediethylester,
4-Brommethyl-4'-brombiphenyl,
4-Brommethyl-4'-phenylsulfonylbiphenyl,
4-Brommethyl-4'-cyanbiphenyl,
4-Brommethyl-3'-methyl-4'-brombiphenyl.

Die Aldehyde der Formeln II bzw. III ($Z_1$ bzw. $Z_2$ = CHO) lassen sich aus den Halogenmethylverbindungen durch Umsetzung mit Hexamethylentetramin in Essigsäure (Sommelet-Reaktion) herstellen. Man erhält sie auch durch Nachsulfierung von Biphenylaldehyden mit Oleum gemäß der in der DE-OS 2 525 681 beschriebenen Arbeitsweise. Ein weiterer Weg zu den Biphenylaldehydsulfonsäuren besteht in der Umsetzung von halogenierten Biphenylaldehyden mit Natriumsulfit.

Geeignete Biphenylaldehyde der Formeln II und III sind:

Biphenyl-4-aldehyd-4'-sulfonsäure, Biphenyl-4-aldehyd-3-sulfonsäure,
Biphenyl-4-aldehyd-3,4'-disulfonsäure, Biphenyl-4-aldehyd, 3,4-Dichlorbiphenyl-4'-aldehyd,
4-Methylbiphenyl-4'-aldehyd-3,3'-disulfonsäure,
3-Chlorbiphenyl-4'-aldehyd-4-sulfonsäure,
4-Brombiphenyl-4'-aldehyd, 4-Cyanbiphenyl-4'-aldehyd,
3-Methyl-4-brombiphenyl-4'-aldehyd.

Die Verbindungen der Formel I können auch durch Umwandlung der Substituenten eines die Grundstruktur der Verbindungen bereits aufweisenden Moleküls in Substituenten, die unter die obige Definition der Formel I fallen, hergestellt werden. Ferner können auch in den Verbindungen der Formel I Substituenten in andere unter dieselbe Definition fallende Substituenten umgewandelt werden.

Die erfindungsgemäßen Verbindungen zeigen in gelöstem oder feinverteiltem Zustand eine mehr oder weniger ausgeprägte Fluoreszenz. Sie lassen sich daher zum optischen Aufhellen synthetischer oder natürlicher hochmolekularer Materialien verwenden.

Unter synthetischen organischen hochmolekularen Materialien sind Polymerisations-, Polykondensations- und Polyadditionsprodukte sowie deren Nachbehandlungsprodukte zu verstehen, z. B.

Polymerisate auf Basis von $\alpha,\beta$-ungesättigten Carbonsäuren, Dicarbonsäuren, Carbonsäureestern, Amiden, Nitrilen, von Olefinkohlenwasserstoffen, von halogenierten oder arylgruppenhaltigen Olefinkohlenwasserstoffen (wie Polyethylen, Polypropylen, Polybutadien, Polyvinylchlorid, Polyvinylidenchlorid, Polyacrylnitril, Polystyrol, Polyacrylsäurederivate und Mischpolymerisate aus zwei oder mehreren der obengenannten polymerisationsfähigen Monomeren), Polykondensate auf Basis bi- oder polyfunktioneller Verbindungen mit kondensationsfähigen Gruppen, deren Homo- und Mischkondensationsprodukte (wie Polyester, Polyamide, Maleinatharze, Polycarbonate, Siliconharze, Phenol- oder Melaminformaldehydharze u. a.), Polyadditionsprodukte, wie vernetzte oder unvernetzte Polyurethane, sowie Epoxidharze.

Als halbsynthetische organische Materialien seien z. B. Celluloseester und -ether, Nitrocellulose, regenerierte Cellulose und Kunststoffe auf Caseinbasis genannt.

Optisch aufhellbare natürliche hochmolekulare organische Materialien sind z. B. Proteinmaterialien, wie Wolle, Seide und Leder; Cellulosematerialien, wie Baumwolle, Papier, Holzmassen in feiner Verteilung; ferner Kautschuk, Guttapercha oder Balata.

Die optisch aufzuhellenden organischen Materialien können in den verschiedensten Verarbeitungszuständen als Rohstoffe, Halbfabrikate oder Fertigfabrikate vorliegen, z. B. als Pulver, Schnitzel, Granulate, Schaumstoffe, Lacke, Dispersionen, Formkörper, wie z. B. Folien, Platten, Filme, Bänder, Fäden, Fasern, z. B. in Form von endlosen Fäden, Stapelfasern, Flocken, Garnen, Strangwaren, Zwirnen, Faservliesen, Filzen, Watten, textilen Geweben, Verbundstoffen und Gewirken, weiterhin auch als Kitte, Pasten, Wachse, Kleb- und Spachtelmassen usw.

Die erfindungsgemäßen Verbindungen werden bevorzugt für die optische Aufhellung von Fasermaterialien und Kunststoffen verwendet. Besonders geeignet sind die in Wasser anionisch löslichen erfindungsgemäßen Verbindungen zur optischen Aufhellung von nativen und regenerierten Cellulosefasern, von Wolle und synthetischen Polyamidfasern.

Besonders gute Aufhelleffekte erhält man zuweilen auch dann, wenn die erfindungsgemäßen Verbindungen mit anderen optischen Aufhellern kombiniert werden. Solche Kombinationen sind insbesondere dann von Interesse, wenn man Nuancenverschiebungen der Aufhelleffekte erzielen will.

Die erfindungsgemäßen Verbindungen der allgemeinen Formel I können auch Waschmitteln, die die üblichen Füll- und Hilfsstoffe enthalten, zugesetzt werden. Sie zeichnen sich insbesondere dadurch aus, daß sie in Gegenwart von oxidativen und reduktiven Bleichmitteln, z. B. Wasserstoffperoxid, Natriumhypochlorit und Natriumchlorit, ohne Beeinträchtigung der optischen Aufhellwirkung eingesetzt werden können.

Lösungen der Verbindungen der Formel I sind auch geeignet zur Erzeugung von kohärenter frequenzveränderlicher monochromatischer Strahlung (Laserlicht) mittels eines Farbstofflasers, der aus einem Reservoir für die Farbstofflösung und einer damit verbundenen Energiequelle besteht, die in der Lage ist, die Farbstofflösung zu einer Emission anzuregen, wobei die erzeugte Strahlung im Wellenlängenbereich von 390 bis 440 nm liegt.

Ein Laser ist eine Lichtverstärkungseinrichtung, mit deren Hilfe es möglich ist, kohärentes monochromatisches Licht einer hohen spektralen und geometrischen Intensitätsdichte zu erzeugen. Der Laser besteht aus einem optischen Resonator, der in einer dünnwandigen Quarz-Zelle das flüssige laseraktive Material enthält. Die Zelle ist gewöhnlich Teil eines geschlossenen Systems, durch welches die Farbstofflösung, während der Laser in Funktion ist, im Kreislauf gepumpt wird. Das aktive Medium kann ebenfalls in Form eines Flüssigkeitsstrahls vorliegen, der aus einer Düse senkrecht zur optischen Achse austritt und den Resonator durchquert. In beiden Anordnungen wird eine lokale Überhitzung vermieden, die zu optischen Inhomogenitäten führen würde.

Die Anregung der Farbstoffe erfolgt mit Hilfe von Energiequellen, mittels Elektronen oder Licht, wobei der Farbstofflaser auch durch einen Gaslaser, beispielsweise einen Stickstoff-, Argon- oder Kryptonlaser angeregt werden kann.

Die Anregung, auch als optisches Pumpen bezeichnet, bewirkt, daß Molekülelektronen des Laserfarbstoffes aus dem Grundzustand auf einen hohen Energiezustand angehoben werden, von dem aus ein Strahlungsübergang erfolgt. Übertrifft die Zahl der im angeregten Zustand befindlichen Moleküle diejenige der in tieferen Zuständen befindlichen Moleküle, so erfolgen stimulierte Übergänge, durch die das Licht im optischen Resonator verstärkt wird.

Ist einer der Laserspiegel partiell lichtdurchlässig, so tritt ein Teil der Strahlung in Form eines Laserstrahls aus der Apparatur aus. Besonders leicht anzuregende Farbstoffe zeigen bei sehr effektiver Anregung die Erscheinung der Superradianz. Diese kann z. B. beobachtet werden, wenn eine Quarzküvette mit der Lösung eines solchen Farbstoffes in den Strahl eines Stickstofflasers gestellt wird. Die Lösung sendet dann, ohne daß sie sich zwischen Resonatorspiegeln befindet, ähnlich wie beim Laser, Licht in eine Vorzugsrichtung aus.

Ein wesentlicher Vorteil des Farbstofflasers im Vergleich zum Festkörper- oder Gaslaser ist dessen Fähigkeit, eine frequenzveränderliche Laserstrahlung zu liefern. Wegen der Fluoreszenzbandbreite der eingesetzten Farbstoffe können Farbstofflaser durch Einfügen eines frequenzselektiven Elements, z. B. eines Reflexionsgitters, Prismas oder doppelbrechenden Filters, so abgestimmt werden, daß Laserlicht bei jeder gewünschten Wellenlänge innerhalb der gesamten Fluoreszenzbande des Farbstoffes emittiert wird.

Obwohl bereits eine Vielzahl von geeigneten Farbstoffen vorgeschlagen wurde, besteht in vielen Bereichen des sichtbaren Wellenlängenbereiches und vor allem im nahen UV trotzdem noch ein erheblicher Mangel an Verbindungen, die einen sehr hohen Wirkungsgrad des Lasers ergeben (s. Optics Communications 24,1 — S. 33 [Januar 1978], und Optics Communications 24,3 — S. 251 [März 1978]). Darüber hinaus war es bisher nicht möglich, kontinuierliche Laserstrahlung unterhalb von 410 nm zu erzeugen. Die erfindungsgemäßen Verbindungen zeichnen sich bei der Anwendung im Farbstofflaser durch eine außerordentlich hohe Lichtbeständigkeit aus.

Laserlicht frequenzveränderlicher Laser hat in den letzten Jahren eine erhebliche Bedeutung in der Spektroskopie erlangt. Die Laser können eingesetzt werden für analytische Zwecke, hochauflösende Spektroskopie, Fluoreszenzspektroskopie, Absorptionsspektroskopie, Lebensdauermessungen, Fotoionisation und bei der Spektroskopie negativer Ionen. Sie haben ferner eine große technische Bedeutung in der Informationstechnik, im Umweltschutz und für die Isotopentrennung.

Beispiel 1

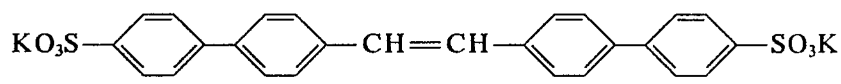

In einem Reaktionsgefäß werden unter Ausschluß von Feuchtigkeit und Luftsauerstoff 40,0 g Kalium-tert.-butylat (ca. 90%ig) in 300 ml Dimethylformamid (wasserfrei) suspendiert. Bei einer Reaktionstemperatur von 40—50°C wird innerhalb von 1 Std. eine filtrierte Lösung aus 40 g biphenyl-4-aldehyd-4'-sulfonsaurem Kalium (ca. 80%ig) in 1100 ml Dimethylformamid, die mit einer Lösung aus 68 g 4-Diethoxyphosphonomethylbiphenyl-4'-sulfonsäureethylester in 100 ml Dimethylformamid verrührt wurde, zugetropft. Es wird 2 Std. bei 40—50°C gerührt, bis im Dünnschichtchromatogramm keine der Ausgangsverbindungen nachgewiesen werden kann. Unter Zusatz von 300 ml Wasser wird ca. 2 Std. bis zur Entfärbung der Reaktionsmischung auf 80—90°C erhitzt. Nach Abkühlen, Absaugen, Waschen mit wenig Wasser und Trocknen im Vakuum bei 80°C wird die Verbindung der oben angegebenen Formel als farbloses kristallines Produkt erhalten. Ausbeute: 56,8 g = 62,5% der Theorie.

Zur Überführung in die leichter löslichen Ammoniumsalze erhitzt man das Kaliumsalz mit halbkonzentrierter Ammoniaklösung, mit Triethanolamin oder mit Tris-2-hydroxyethoxyethylamin.

Das als Ausgangsmaterial verwendete biphenyl-4-aldehyd-4'-sulfonsaure Kalium wurde in folgender Weise gewonnen:

182 g Biphenyl-4-aldehyd wurden portionsweise in 400 g 20%ig. Oleum so eingetragen, daß die Temperatur langsam von 25°C auf 50°C ansteigt. Die Temperatur wird 3 Std. bei 50°C gehalten und die Reaktionsmischung dann auf 1000 g Eis und 30 g Kaliumchlorid ausgetragen. Das biphenyl-4-aldehyd-4'-sulfonsaure Kalium fällt als flockiger Niederschlag aus. Das Rohprodukt wird unter Zusatz von A-Kohle aus Wasser umkristallisiert. Ausbeute: 258,3 g = 85% der Theorie.

Der als Ausgangsmaterial verwendete 4-Diethoxyphosphonomethylbiphenyl-4'-sulfonsäureethylester wurde in folgender Weise hergestellt:

1. Stufe: 4-Hydroxymethylbiphenyl-4'-sulfonsäure

166 g biphenyl-4-aldehyd-4'-sulfonsaures Kalium (80%ig) werden in 600 ml Wasser suspendiert und mit 100 ml 10%iger Natronlauge versetzt. Unter Stickstoff wird eine Lösung aus 6,2 g Natriumborhydrid in 300 ml Wasser und 30 ml 10%iger Natronlauge portionsweise zugesetzt. Unter starker Wasserstoffentwicklung steigt die Temperatur auf 30°C. Es wird 14 Std. bei Raumtemperatur gerührt. Nach vollständiger Reduktion wird vorsichtig mit konz. Salzsäure angesäuert und die Suspension zur Zerstörung des Borkomplexes und zur Bildung der Säure 3 Std. auf 80°C erhitzt. Nach Abkühlen saugt man die 4-Hydroxymethylbiphenyl-4'-sulfonsäure ab, wäscht sie mit wenig Wasser und trocknet sie im Vakuum bei 100°C. Ausbeute: 113 g = 93% der Theorie.

2. Stufe: 4-Chlormethylbiphenyl-4'-sulfonsäurechlorid

113 g 4-Hydroxymethylbiphenyl-4'-sulfonsäure werden in 800 ml Chlorbenzol suspendiert und zur vollständigen Entfernung von Wasser 250 ml Chlorbenzol abdestilliert. Dann wird auf 80—90°C abgekühlt und nach Zusatz von 3 ml Dimethylformamid und 1 g Phosphorpentachlorid werden innerhalb von 2 Std. 150 ml Thionylchlorid zugetropft. Die Temperatur wird 14 Std. bei 85—90°C gehalten. Das Lösungsmittel und überschüssiges Thionylchlorid werden im Vakuum fast bis zur Trockne abdestilliert. Der Rückstand wird mit 900 ml Methylcyclohexan heiß extrahiert, wobei Salze und nicht umgesetzte Sulfonsäure als Rückstand verbleiben, während das Sulfochlorid sich aus Methylcyclohexan beim Abkühlen als farblose kristalline Verbindung abscheidet. Ausbeute: 100,6 g = 78% der Theorie, Schmelzpunkt: 110—112°C.

3. Stufe: 4-Chlormethylbiphenyl-4'-sulfonsäureethylester

98 g 4-Chlormethylbiphenyl-4'-sulfonsäurechlorid werden unter Feuchtigkeits- und Sauerstoffausschluß in 400 ml Tetrahydrofuran (wasserfrei) gelöst und mit einem 10%igen Überschuß an Natriumalkoholat, hergestellt aus 8 g Natrium und 1000 ml absolutem Alkohol, portionsweise versetzt, so daß die Reaktionstemperatur unter Eiskühlung bei 10—15°C gehalten wird. Nach einstündigem Rühren bei Raumtemperatur wird das Lösungsmittelgemisch im Vakuum bei 50°C abgezogen. Der Rückstand wird mit 1200 ml Di-sek.-butylether (wasserfrei) heiß extrahiert. Nach Abkühlen, Absaugen und Trocknen im Vakuum bei 50°C erhält man 68,5 g 4-Chlormethylbiphenyl-4'-sulfonsäureethylester. Ausbeute: 68%, Schmelzpunkt: 112—116°C.

4. Stufe: 4-Diethoxyphosphonomethylbiphenyl-4'-sulfonsäureethylester

48,5 g 4-Chlormethylbiphenyl-4'-sulfonsäureethylester werden unter Feuchtigkeits- und Sauerstoffausschluß in 100 ml wasserfreiem Xylol suspendiert. Innerhalb von 2 Std. werden bei 130—140°C beginnend 75 g Triethylphosphit unter gleichzeitiger Abdestillation des entstehenden Ethylchlorids und von Xylol und Triethylphosphit zugetropft, so daß die Innentemperatur langsam auf 165—170°C ansteigt. Die Reaktionsmischung wird 7 Std. bei dieser Temperatur gehalten. Überschüssiges Triethylphosphit wird bei 160°C und 20 mbar abdestilliert. Das als Rückstand verbleibende Rohöl (68 g) wird in 100 ml Dimethylformamid aufgenommen und in dieser Form mit biphenyl-4-aldehyd-4'-sulfonsaurem Kalium umgesetzt.

Beispiel 2

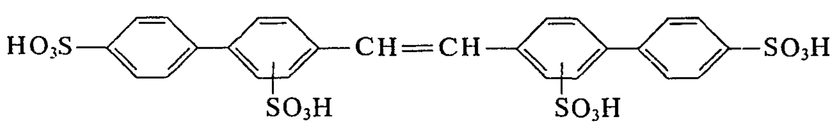

10 g der gemäß Beispiel 1 hergestellten Verbindung werden in 50 ml 20%ig. Oleum bei 0—10°C eingetragen und 30 Min. bei 5°C verrührt. Innerhalb von 1 Std. wird die Temperatur auf 25°C erhöht. Nach Austragen auf ein Gemisch von Eis und Wasser wird mit Calciumhydroxid neutralisiert, das entstehende Calciumsulfat abfiltriert und gründlich mit Wasser gewaschen. Das Filtrat wird mit A-Kohle geklärt und das Calciumsalz an einem stark sauren Ionenaustauscher in die freie Sulfonsäure überführt.

In ähnlicher Weise wie in den vorstehenden Beispielen beschrieben, können die in der nachfolgenden Tabelle aufgeführten Diphenylstilbensulfonsäuren der Formel

$$A-CH=CH-A'$$

dargestellt werden.

| A | A′ |
|---|---|
| 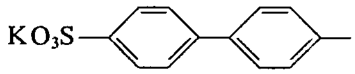 | 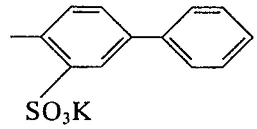 |
| 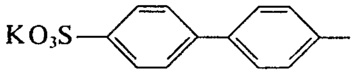 | 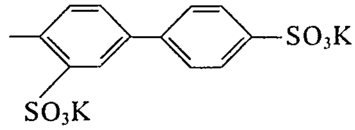 |
| 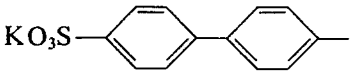 | 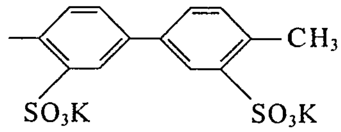 |
| 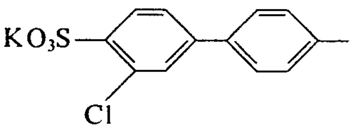 | 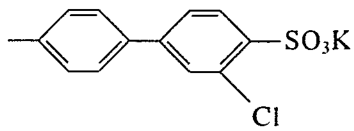 |
| 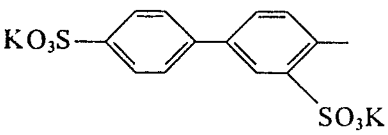 | 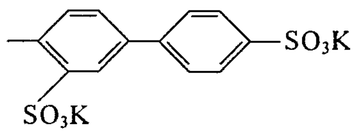 |
| 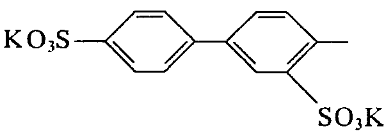 | 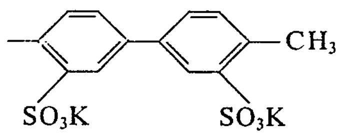 |
| 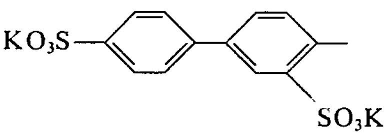 | 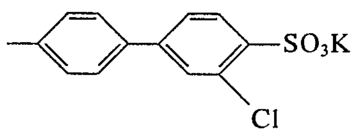 |
| 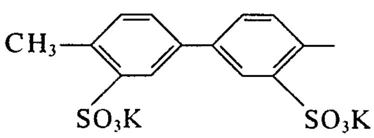 | 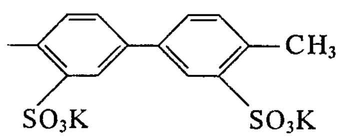 |
| 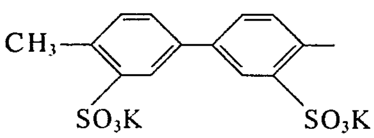 | 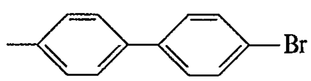 |
| 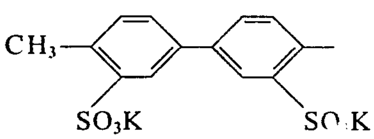 | 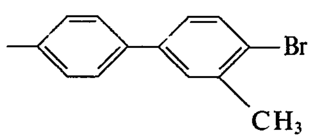 |
| 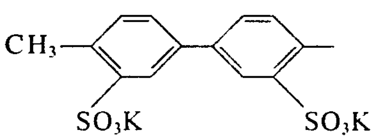 | 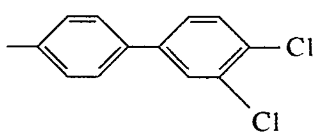 |

Fortsetzung

| A | A′ |
|---|---|
| 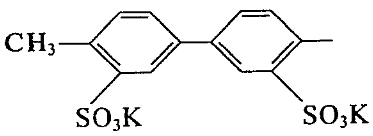 | 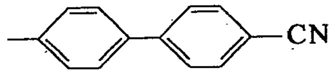 |
| 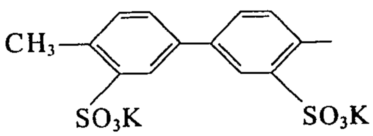 | 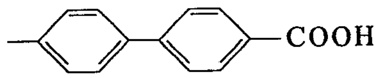 |
| 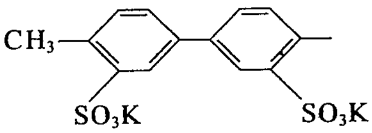 | 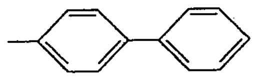 |
| 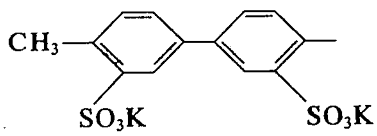 | 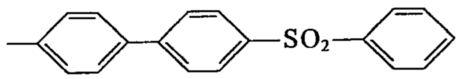 |
| 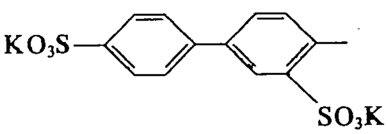 | 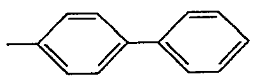 |
| 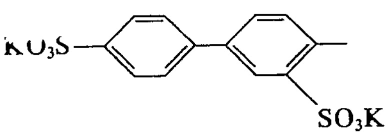 | 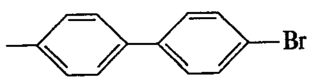 |
| 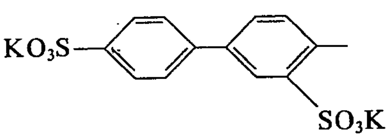 | 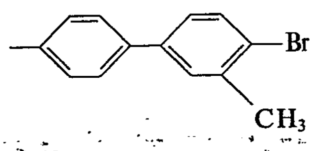 |
| 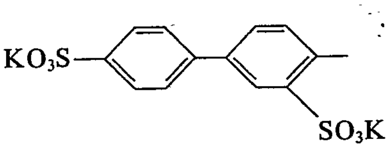 | 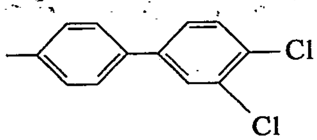 |
| 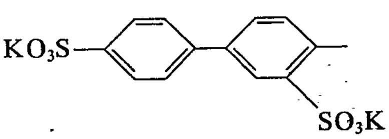 | 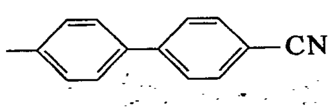 |
| 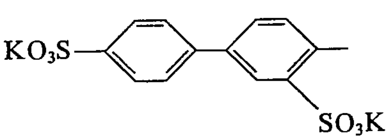 | 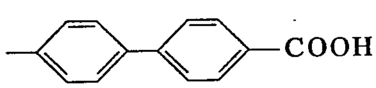 |
| 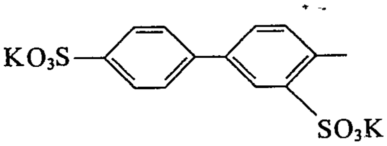 | 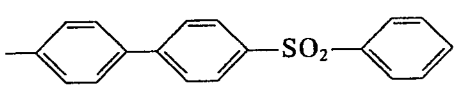 |
| 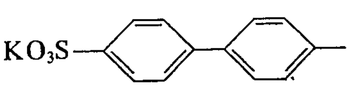 | 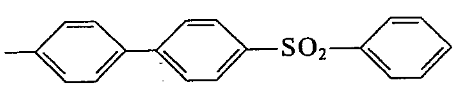 |

Beispiel 3

Ein Polyamidfasergewebe wird im F'ottenverhältnis 1/40 bei 60°C in ein Bad eingebracht, das, bezogen auf das Stoffgewicht, 0,1% d in Beispiel 2 beschriebenen Aufhellers sowie pro Liter 1 g 80%ige Essigsäure und 0,25 g eines A agerungsproduktes von 30—35 Mol Ethylenoxid an 1 Mol technischen Stearylalkohol enthält. Man erwärmt innerhalb von 30 Min. auf Kochtemperatur und hält während 30 Min. beim Sieden. Nach dem Spülen und Trocknen zeigt das Polyamidgewebe einen guten Aufhelleffekt.

Beispiel 4

Die nach den Angaben des Beispiels 1 hergestellte Verbindung wurde in einer Konzentration von $1,5 \times 10^{-3}$ Mol/l in einem Gemisch aus 50% Methanol und 50% Wasser gelöst. In einer Apparatur gemäß Fig. 1 wurde diese Lösung aus einem Reservoir durch die Farbstoffzelle (3) gepumpt. Das Durchstimmen der Wellenlänge erfolgte mittels eines Reflexionsgitters (2) mit Schrittmotorantrieb (1). Die Aufnahme des Laserspektrums erfolgte über einen Photomultiplier (7), der spektral geeicht war, die Wellenlängenkalibrierung über einen Monochromator (6). Zur Leistungsmessung wurde der Photomultiplier durch einen Thermopile-Meßkopf mit zugehörigem Meßverstärker ersetzt. Die Intensität in Prozent der Pumpleistung wird zugleich in kW angegeben, da die Ausgangsleistung des Pumplasers 100 kW betrug. In Fig. 1 bedeuten weiterhin: (4) $N_2$-Laser und (5) 30% Auskoppelspiegel.

Der verwendete Stickstofflaser hatte eine Wellenlänge von 337 nm, eine Pulsfrequenz von 100 Hz, eine Pulsbreite von 7 nsec und eine Pulsspitzenleistung von 100 kW.

Die Abhängigkeit der Laserleistung von der Wellenlänge ist in Fig. 2 wiedergegeben. Als Vergleichssubstanz diente der bekannte Laserfarbstoff POPOP (1,4-Bis-[2-(5-phenyloxazolyl)]-benzol), dessen Laseraktivität in Optics Communications 24,1 — S. 33 (Januar 1978) beschrieben ist. Die erfindungsgemäße Verbindung zeigt eine höhere Ausgangsleistung über einen erweiterten Spektralbereich.

Erläuterung zu Fig. 2: I—Intensität (in % Pumpleistung); L—Wellenlänge (nm); A—Laserleistungskurve des Fluoreszenzfarbstoffes gemäß Beispiel 1; Laserleistungskurve des Laserfarbstoffes POPOP; Konzentration $1,5 \times 10^{-3}$ molar; Lösungsmittel 1/1, $H_2O/CH_3OH$; Stickstofflaser 100 kW/100 Hz; Farbstofflaseraufbau wie in Fig. 1.

Beispiel 5

Der nach den Angaben des Beispiels 1 hergestellte Fluoreszenzfarbstoff wurde in einer Konzentration von $1,35 \times 10^{-3}$ Mol/l in Ethylenglykol gelöst. In einer Farbstofflaseranordnung gemäß Fig. 3 wurde diese Lösung mittels eines Düsenstrahls in den Laserresonator eingebracht. Der als Pumplaser zur kontinuierlichen Anregung verwendete Kryptonlaser (15) hatte eine Wellenlänge von 351/356 nm und eine maximale Ausgangsleistung von 3,5 W. Das Durchstimmen der Wellenlänge erfolgte durch ein doppelbrechendes Filter (11). Die Nutzleistung wurde mittels einer Quarzplatte (13) ausgekoppelt, die unter einem geeigneten Winkel in den Laserresonator eingefügt wurde. Die Aufnahme des Laserspektrums wurde punktweise durch Drehen des frequenzselektiven doppelbrechenden Filters vorgenommen, wobei die Ausgangsleistung bei einer eingestellten Wellenlänge mit einem Thermopile-Leistungsmesser ermittelt wurde. Die Ausgangsleistung ist in mW angegeben.

In Fig. 3 bedeuten weiterhin: (9) durchstimmbarer Ausgangsstrahl; (10) Faltfilter (50 mm Radius); (12) Endspiegel (plan); (14) Farbstoff-Jet (unter Brewster-Winkel); (16) Pumpspiegel; (17) Endspiegel (50 mm Radius).

Die Abhängigkeit der Laserleistung von der Wellenlänge ist in Fig. 4 wiedergegeben. Als Vergleichsfarbstoff dient Stilben 3, dessen Laseraktivität in Optics Communications 24,3 — S. 251 (März 1978) beschrieben ist. Die erfindungsgemäße Verbindung zeigt eine höhere Ausgangsleistung über einen wesentlich zum nahen UV erweiterten Spektralbereich. Die erfindungsgemäße Farbstoffklasse erlaubt somit zum ersten Mal einen cw-Betrieb im Spektralbereich unter 400 nm.

Ähnlich gute Ergebnisse erhält man, wenn man anstelle der oben angegebenen Verbindung Fluoreszenzfarbstoffe einsetzt, deren Formeln im Anschluß an Beispiel 2 aufgeführt wurden.

Erläuterung zu Fig. 4: A—Ausgangsleistung; L—Wellenlänge; Laserleistungskurve des Fluoreszenzfarbstoffes gemäß Beispiel 1; Laserleistungskurve von Stilben 3 (3—5% Auskopplung); Konzentration $1,3 \times 10^{-3}$ molar; Lösungsmittel Ethylenglycol; Auskopplung ca. 5%; Pumpleistung 3,5 Watt UV.

**Patentansprüche**

1. Fluoreszenzfarbstoffe der Formel

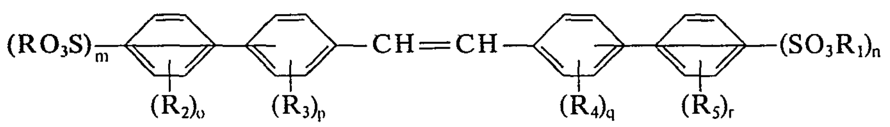 (I)

worin

| | |
|---|---|
| R und $R_1$ | unabhängig voneinander für Wasserstoff, ein salzbildendes Kation, einen Alkylrest mit 1 bis 8 Kohlenstoffatomen oder einen Aralkylrest, |
| $R_2$ bis $R_5$ | unabhängig voneinander für Wasserstoff, Alkyl, Trifluormethyl, Alkoxy, Aralkoxy, Alkenyloxy, Halogen, die Carboxyl-, Cyan-, Alkylsulfon-, Arylsulfon-, Carbonamid-, Sulfonamid- oder die Carbonsäureestergruppe stehen, |
| m und n | unabhängig voneinander 0, 1 und 2 bedeuten, wobei die Summe von m+n mindestens gleich 2 sein muß, und |
| o, p, q und r | unabhängig voneinander für 0, 1 und 2 stehen. |

2. Fluoreszenzfarbstoffe gemäß Anspruch 1 der Formel

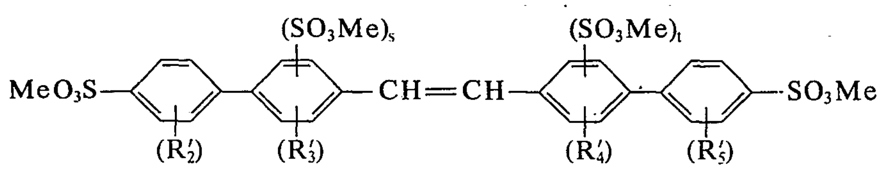

worin

| | |
|---|---|
| Me | für Wasserstoff, Natrium, Kalium oder den gegebenenfalls substituierten Ammoniumrest, |
| $R_2'$ bis $R_5'$ | unabhängig voneinander für Wasserstoff, $C_1$- bis $C_4$-Alkyl, Benzyloxy, Cyan sowie eine Carboxy-, Carbonsäureester- oder Carbonamidgruppe stehen und |
| s und t | 0 oder 1 bedeuten. |

3. Fluoreszenzfarbstoffe gemäß Anspruch 1 der Formel

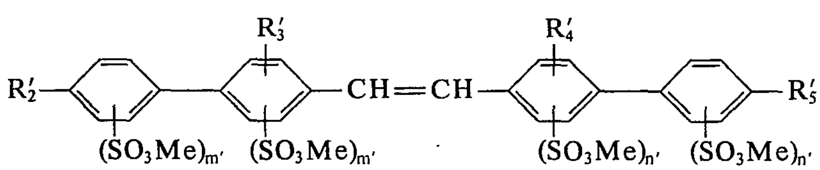

worin

| | |
|---|---|
| Me und $R_2'$ bis $R_5'$ | die gleiche Bedeutung wie in Anspruch 2 haben und |
| m' und n' | für 0 oder 1 stehen, wobei die Summe von m' und n' mindestens 2 sein muß. |

4. Fluoreszenzfarbstoff gemäß Anspruch 1 der Formel

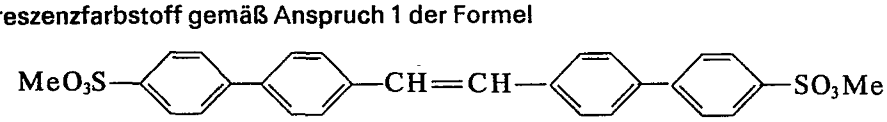

in welcher

| | |
|---|---|
| Me | die gleiche Bedeutung wie in Anspruch 2 hat. |

5. Verfahren zur Herstellung von Fluoreszenzfarbstoffen gemäß Anspruch 1, dadurch gekennzeichnet, daß man eine Verbindung der Formel

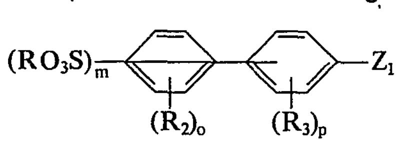

mit einer Verbindung der Formel

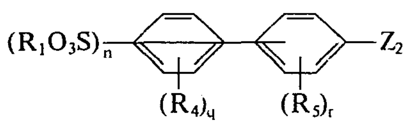

(III)

worin

die Reste R bis $R_5$ und die Indizes m, n, o, p, q, r die gleiche Bedeutung wie in Formel I haben und $Z_1$ und $Z_2$ wechselseitig für die Aldehyldgruppe oder einen Rest der Formeln

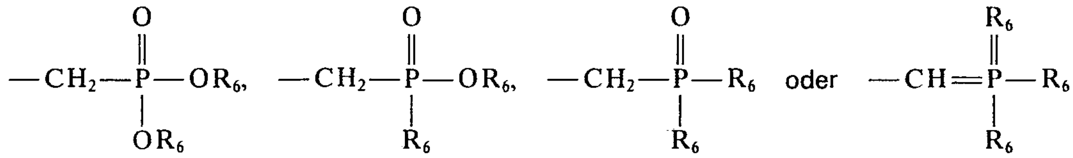

$R_6$ = Alkyl $C_{1-6}$, Aryl, Aralkyl, Cycloalkyl
stehen,

in Gegenwart einer stark basischen Alkaliverbindung und in Gegenwart eines vorzugsweise hydrophilen stark polaren Lösungsmittels kondensiert.

6. Verwendung von Fluoreszenzfarbstoffen der Ansprüche 1 bis 3 zum Weißtönen organischer Materialien.

7. Verwendung von Fluoreszenzfarbstoffen gemäß den Ansprüchen 1 bis 4 zur Erzeugung einer kohärenten Laseremission in einem Wellenlängenbereich von 390 bis 440 nm.

**Claims**

1. Fluorescent dyestuffs of the formula

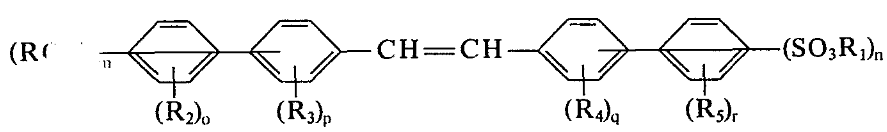

wherein

| | |
|---|---|
| R and $R_1$ | independently of one another represent hydrogen, a salt-forming cation, an alkyl radical with 1 t 8 carbon atoms or an aralkyl radical, |
| $R_2$ to $R_5$ | independently of one another represent hydrogen, alkyl, trifluoromethyl, alkoxy, aralkoxy, alkenyloxy, halogen, the carboxyl, cyano, alkylsulphonyl, arylsulphonyl, carboxamide or sulphonamide group or a carboxylic acid ester group, |
| m and n | independently of one another denote 0, 1 or 2 and the sum of m+n must be at least 2, and |
| o, p, q and r | independently of one another represent 0, 1 or 2. |

2. Fluorescent dyestuffs according to Claim 1 of the formula

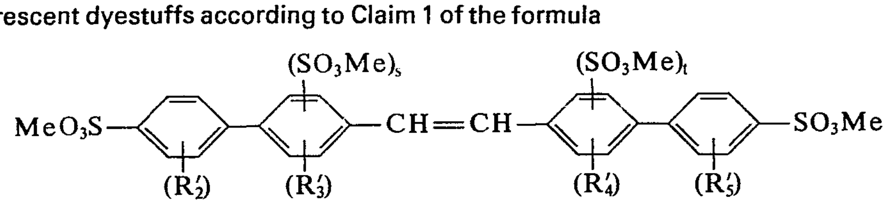

wherein

| | |
|---|---|
| Me | represents hydrogen, sodium, potassium or an optionally substituted ammonium radical, |
| $R_2'$ to $R_5'$ | independently of one another represent hydrogen, $C_1$- to $C_4$-alkyl, benzyloxy, cyano, or a carboxyl, carboxylic acid ester or carboxamide group and |
| s and t | denote 0 or 1. |

3. Fluorescent dyestuffs according to Claim 1 of the formula

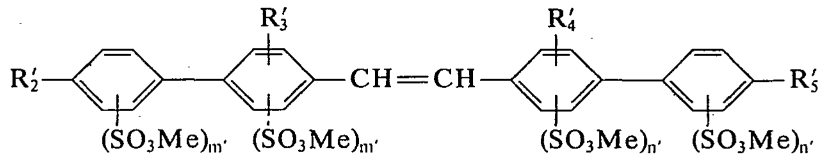

wherein

| | |
|---|---|
| Me an $R_2'$ to $R_5'$ | have the same meaning as in Claim 2 and |
| m' and n' | represent 0 or 1 and the sum of m' and n' must be at least 2. |

4. A fluorescent dyestuff according to Claim 1 of the formula

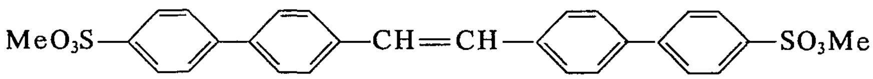

in which

| | |
|---|---|
| Me | has the same meaning as in Claim 2. |

5. Process for the preparation of fluorescent dyestuffs according to Claim 1, characterised in that a compound of the formula

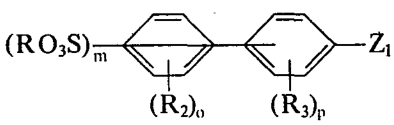

is subjected to a condensation reaction with a compound of the formula

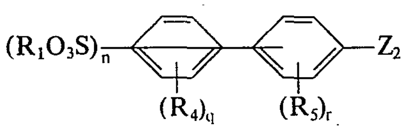

wherein

the radicals R to $R_5$ and the indices m, n, o, p, q and r have the same meaning as in Claim 1 and $Z_1$ and $Z_2$ reciprocally represents the aldehyde group or a radical of the formulae

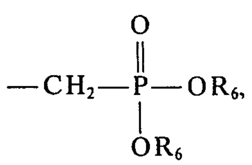 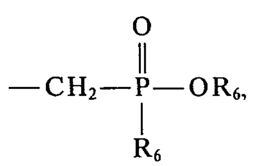 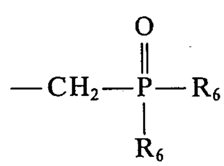 or 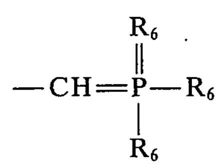

$R_6$ = alkyl $C_{1-6}$, aryl, aralkyl or cycloalkyl

in the presence of a strongly basic alkali metal compound and in the presence of a preferably hydrophilic, strongly polar solvent.

6. Use of fluorescent dyestuffs of Claims 1 to 3 for whitening organic materials.

7. Use of fluorescent dyestuffs according to Claims 1 to 4 for the production of a coherent laser emission in a wavelength range of 390 to 440 nm.

**Revendications**

1. Colorants fluorescents de formule:

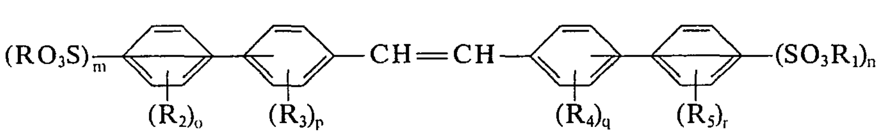

dans laquelle:

R et $R_1$ représent, indépendamment l'un de l'autre, de l'hydrogène, un cation formant un sel, un reste alkyle ayant 1 à 8 atomes de carbone ou un reste aralkyle,

$R_2$ à $R_5$ représentent, indépendamment l'un de l'autre, de l'hydrogène, un reste alkyle, trifluorométhyle, alkoxy, aralkoxy, alcényloxy, un halogène, le groupe carboxyle, cyano, alkylsulfo, arylsulfo, carbonamido, sulfonamido ou le groupe ester d'acide carboxylique,

m et n représentent, indépendamment l'un de l'autre, les nombres 0, 1 et 2, la somme m + n devant au moins être égale à 2, et

o, p, q et r représentent, indépendamment les uns des autres, les nombres 0, 1 et 2.

2. Colorants fluorescents suivant la revendication 1, de formule:

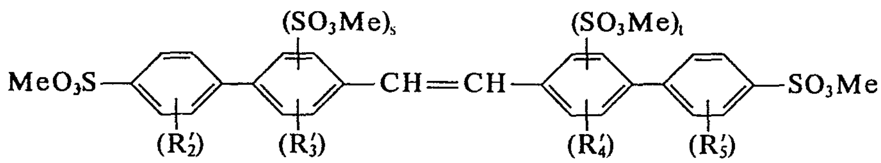

dans laquelle:

Me désigne l'hydrogène, le sodium, le potassium ou le reste ammonium éventuellement substitué,

$R_2'$ à $R_5'$ désignent, indépendamment l'un de l'autre, l'hydrogène, un groupe alkyle en $C_1$ à $C_4$, benzyloxy, cyano ainsi qu'un groupe carboxy, ester d'acide carboxylique ou carbonamido, et

s et t sont égaux à 0 ou à 1.

3. Colorants fluorescents, suivant la revendication 1, de formule:

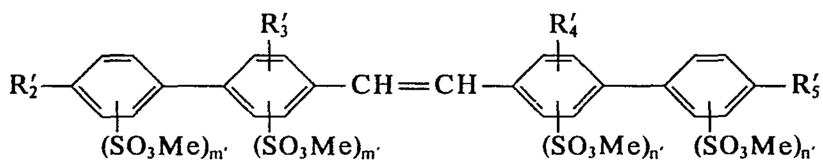

dans laquelle:

Me et $R_2'$ à $R_5'$ ont la même définition que dans la revendication 2 et

m' et n' sont égaux à 0 ou à 1, la somme de m' et n' devant au moins être égale à 2.

4. Colorant fluorescent suivant la revendication 1, de formule:

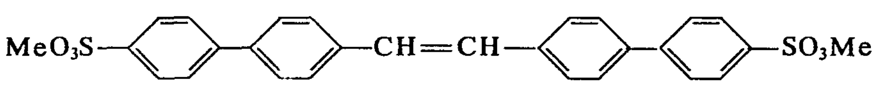

dans laquelle:

Me a la même définition que dans la revendication 2.

5. Procédé de production de colorants fluorescents suivant la revendication 1, caractérisé en ce qu'on condense, en présence d'un composé alcalin fortement basique et en présence d'un solvant fortement polaire de préférence hydrophile, un composé de formule:

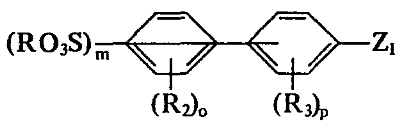

avec un composé de formule:

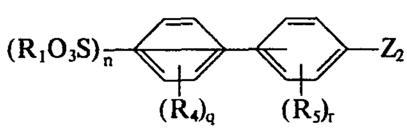

où

les restes R à $R_5$ et les indices m, n, o, p, q, r ont la même définition que dans la revendication 1 et $Z_1$ et $Z_2$ représentent réciproquement le groupe aldéhyde ou un reste de formules:

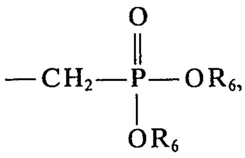, 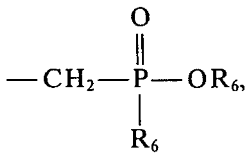, 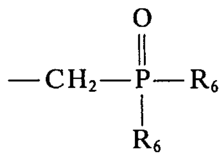 ou 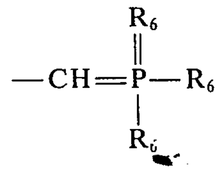

$R_6$ = alkyle en $C_1$ à $C_6$, aryle, aralkyle, cycloalkyle.

6. Utilisation de colorants fluorescents des revendications 1 à 3 pour l'azurage de matières organiques.

7. Utilisation de colorants fluorescents suivant les revendications 1 à 4 pour la production d'une émission laser cohérente dans un intervalle de longueurs d'ondes de 390 à 440 nm.

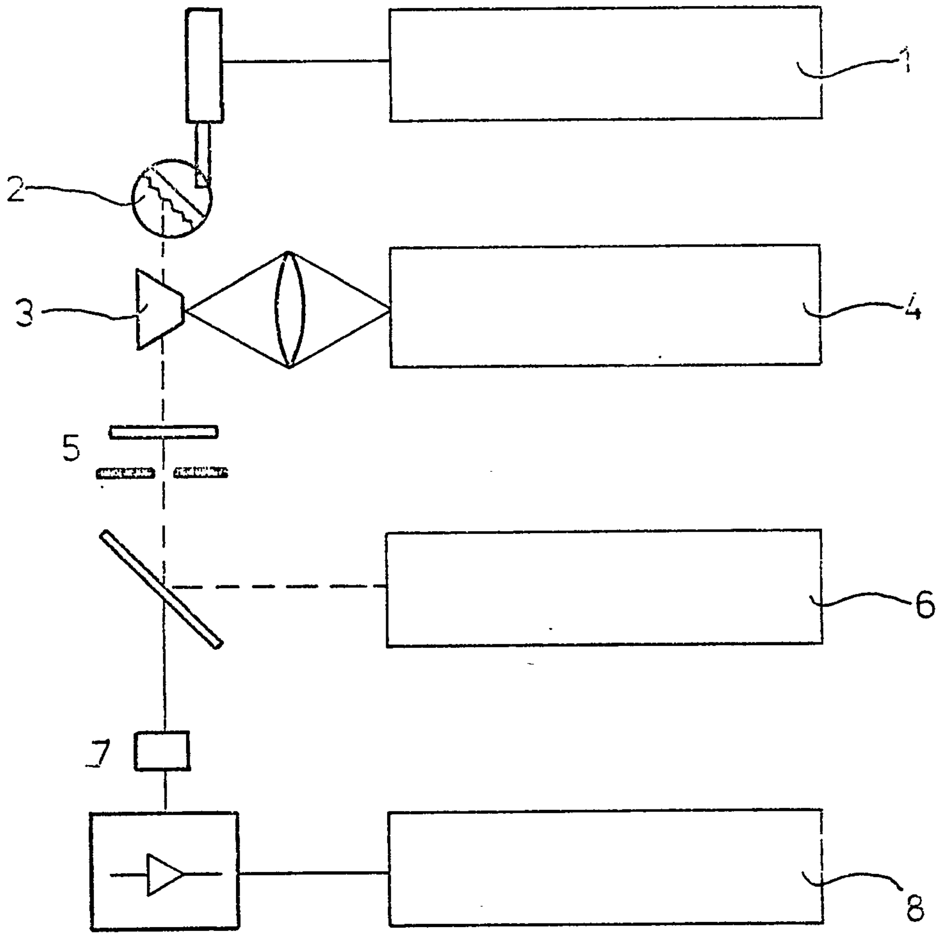


FIG.1

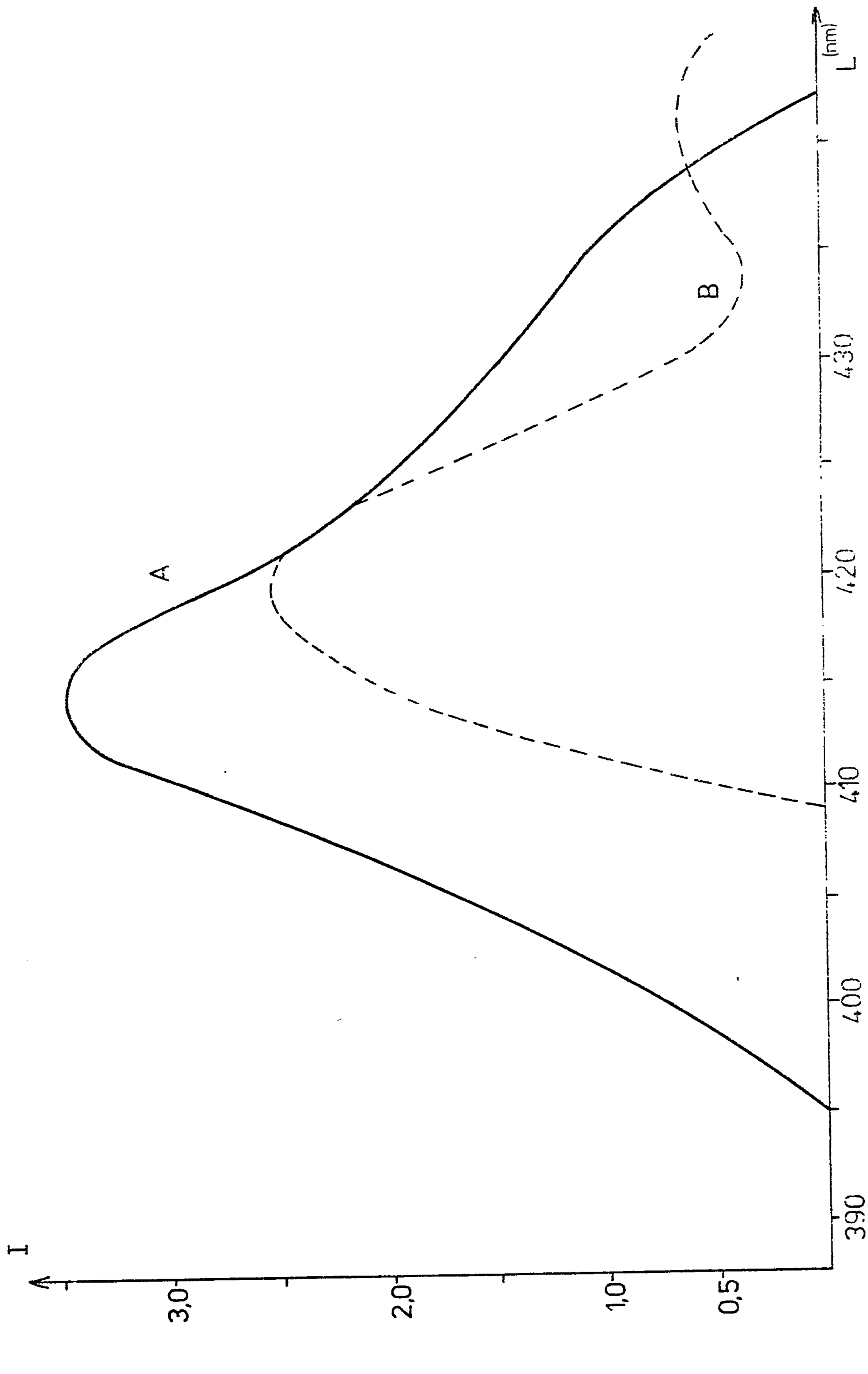


FIG.2

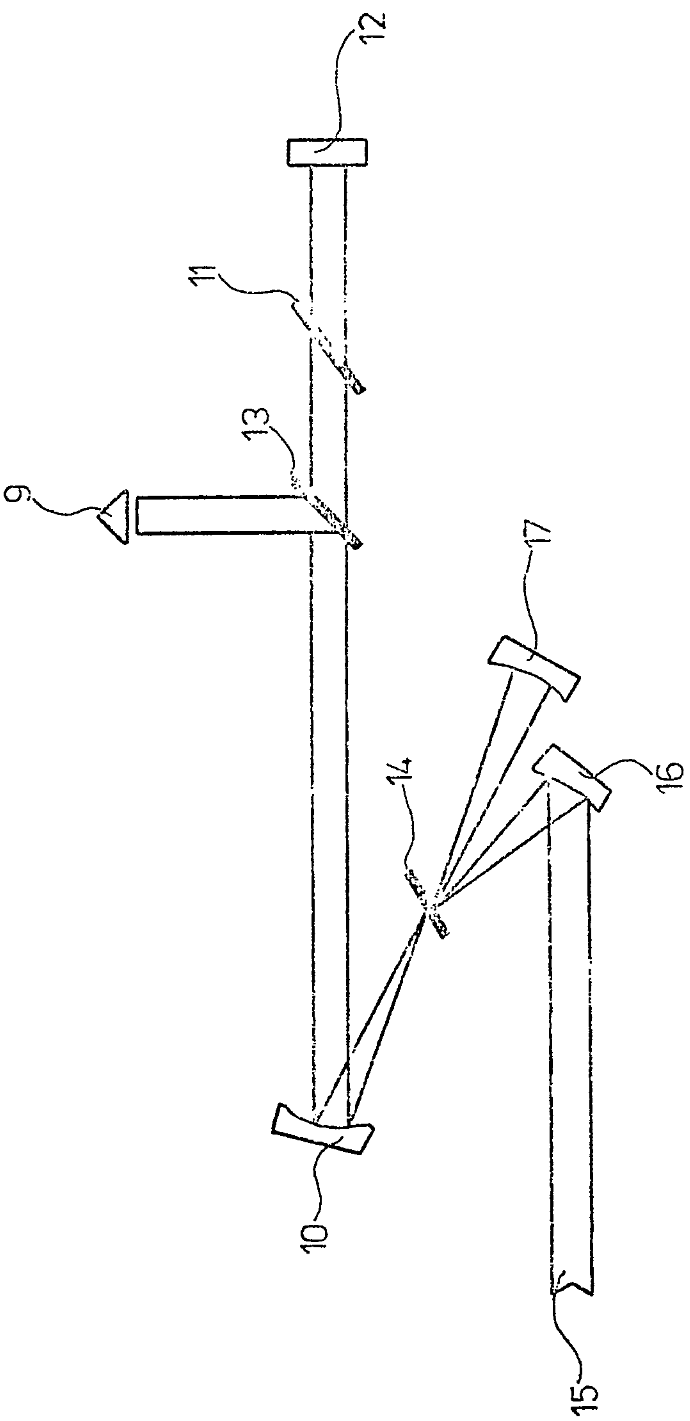


FIG.3

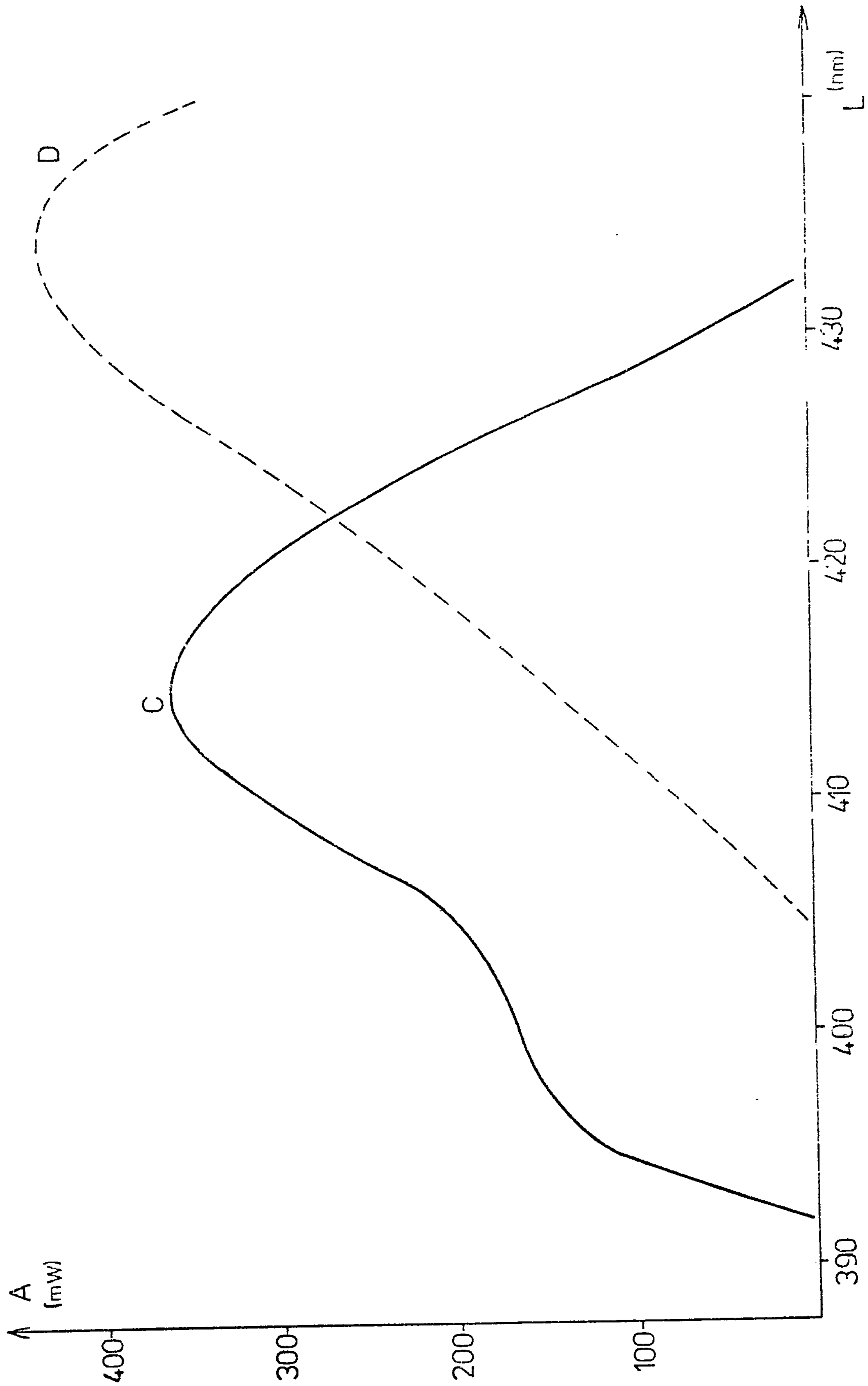


FIG.4